# EUROPEAN PATENT APPLICATION

(11) **EP 3 760 243 A1**
(43) Date of publication of application: **06.01.2021**
(21) Application number: 19184488.5
(22) Date of filing: 04.07.2019
(51) Int. Cl.: A61L 29/12, A61L 29/14, A61L 29/16

(54) **ANTIBACTERIAL ARTICLE COMPRISING A POLYMER MATRIX WITH ALIGNED NANOSCALE FLAKES OR PLATELETS**

(71) Applicant: Dentsply IH AB, 431 21 Mölndal (SE)
(72) Inventor: KÁDÁR, Roland, 436 50 HOVÅS (SE); MIJAKOVIC, Ivan, 431 44 MÖLNDAL (SE); GASKA, Karolina, 412 79 GÖTEBORG (SE); PANDIT, Santosh, 412 82 GÖTEBORG (SE); SVENSSON, Magnus, 445 34 BOHUS (SE)
(74) Representative: AWA Sweden AB

(57) **Abstract**

An article having an antibacterial surface is disclosed, wherein the article comprises a substrate comprising a polymer matrix and a filler material comprising nanoscale flakes or platelets. The article comprises an antibacterial surface having the nanoscale flakes or platelets arranged essentially aligned to each other and extending out from said surface with a length in the range of 0.5-30 microns. The antibacterial surface is produced by processing a mixture of a polymer matrix material and a filler material comprising the nanoscale flakes or platelets by pressing the mixture through a die while heated to a temperature above a melting temperature of the polymer matrix material. Hereby, the nanoscale flakes or platelets become aligned, with their longitudinal directions being oriented in substantially the same direction. A surface of the processed mixture which is oriented essentially perpendicularly to the longitudinal directions of the nanoscale flakes or platelets is then etched or ablated to partly expose the nanoscale flakes or platelets, thereby making the surface antibacterial.

## Description

### Field of the invention

The present invention generally relates to an antibacterial device or article, comprising an antibacterial surface being provided on a surface or a part of a surface of the device/article. The invention also relates to a method for producing such an antibacterial surface.

### Background of the invention

Bacterial infections are a big problem in the society, and there is a general need to reduce the number of bacteria in many situations, such as in tap water, in medical devices, etc.

One way of reducing bacteria is to clean and sterilize equipment, and to use products in a clean way, thereby avoiding contamination. However, despite adherence to sterile guidelines etc, the use of e.g. medical devices, and in particular introduction of medical devices into natural and artificial body openings, implies a risk of bacterial contamination. For example, insertion and maintenance of urinary catheters poses a problem in relation to catheter-associated infections. When medical devices such as catheters are introduced into the human cavity, the normal human defense barrier may be penetrated, which can result in introduction of bacteria, fungi, vira, or tissue-like or multiple organized cells. Urinary tract infection (UTI), for instance, is a problem associated with the use of urinary catheters, and in particular for indwelling catheters, so-called Foley catheters, but also for catheters for intermittent use. It is estimated that almost one-quarter of hospitalized spinal cord-injured patients develop symptomatic UTI during their hospital course. Gram-negative bacilli account for almost 60-70 percent, enterococci for about 25 percent and Candida species for about 10 percent of cases of UTI. It is also well known that persons practicing intermittent urethral catheterization as a daily routine often have problems with symptomatic UTI.

Similar problems are present also in medical irrigation devices, such as devices for anal irrigation, vaginal irrigation, etc.

Thus, cleanliness, sterilization and the like are efficient to reduce the number of bacteria, but is often not sufficient.

Further, it is well known to use antibacterial agents/compounds to kill bacteria, such as antibiotics and the like, that either kills or inhibits growth of bacteria. The antibacterial agents may be administered directly to humans and animals, or be arranged in coatings on medical devices and other devices. However, the use of such antibacterial agents/compounds have several negative effects, such as the development of resistance of bacteria, and unwanted harmful side-effects. There is therefore a general need to limit the use of antibacterial agents/compounds, and in particular antibiotic drugs, to situations where it is of real necessity. Also, due to the increased emergence of bacteria resistance, the antibacterial effect for many known antibacterial agents is decreasing.

In US 2018/320002 by the same applicant, it was shown that a very efficient antibacterial surface could be provided by providing Angstrom scale flakes, such as graphene or graphite flakes, on a substrate in a standing position, and attached to the surface via edge sides of the flakes. Hereby, a great bactericidal effect was obtained on prokaryotic cells, but without harming eukaryotic cells, and it was assumed that this was due to the flakes ability to penetrate into the smaller prokaryotic cells, but not into the larger eukaryotic cells. However, unfortunately this antibacterial surface is cumbersome and costly to produce, and cannot be scaled to industrial manufacturing.

There is therefore a need for new and improved ways of killing bacteria and/or inhibiting growth of bacteria. Specifically, there is a need for antibacterial coatings or surfaces with adequate bactericidal effect, and which can be produced cost-efficiently, and where the production process is scalable to allow for industrial production.

### Summary of the invention

It is a general object of the present invention to fulfil the above-discussed need and alleviate the above-discussed problems.

This object is fulfilled by an antibacterial article and a method in accordance with the appended claims.

According to a first aspect of the invention, there is provided a method for producing an antibacterial surface, comprising the steps:
providing a polymer matrix material;
providing a filler material comprising nanoscale flakes or platelets;
processing a mixture of said polymer matrix material and said filler material by pressing said mixture through a die while heated to a temperature above a melting temperature of said polymer matrix material in such a way that the nanoscale flakes or platelets become aligned, with their longitudinal directions being oriented in substantially the same direction;
providing a surface of the processed mixture which is oriented essentially perpendicularly to the longitudinal directions of the nanoscale flakes or platelets;
etching or ablating the surface to partly expose the nanoscale flakes or platelets, thereby making the surface antibacterial.

In the context of the present application, "antibacterial" is used to indicate an ability to destruct and inhibit growth of bacteria. However, the antibacterial surface may in addition have an ability to destruct and inhibit growth of other microorganisms, such as certain fungi, parasites, and viruses. Thus, the surface may also be referred to as "antimicrobial".

The present invention is based on the realization that industrial manufacturing of defect-free, monolayer graphene or graphene oxide on a scale required for antimicrobial applications is currently not feasible, since chemical and physical routes for producing such pure materials from bulk graphite are too time consuming, laborious, and costly. However, the present inventors have realized that nanoscale flakes or platelets, such as graphite nanoplatelets (GNP), which are considerably cheaper and readily available in bulk quantities, can be processed in a way that would ensure similar antimicrobial effects. Specifically, controlled orientation of graphite nanoplatelets within a polymer has been found to be a relatively easy and cost-effective approach to produce antibacterial surfaces, and these antibacterial surfaces have been shown to be surprisingly efficient in preventing colonization of pathogenic bacteria.

In the present application, "nanoscale flake" and nanoscale platelet" refers to a flake or platelet, i.e. an even or uneven piece of material with one dimension, the thickness, substantially smaller than the other two dimensions (length and height), and where the thickness is of nanoscale or nano dimension, i.e. between 0.01 and 100 nm, and preferably between 0.1 and 50 nm. In preferred embodiments, the flakes or platelets have an average thickness in the range of 0.01-20 nm, and preferably in the range 0.1-15 nm, and more preferably in the range of 0.5-10 nm, and most preferably in the range of 1-5 nm. The flake is not necessarily flat but can assume both plate-like forms as well as various curved shapes.

In one embodiment, the nanoscale flakes or platelets are in the form of so-called GNP, graphite nanoplatelets. GNPs are semi-metallic graphite crystals that are typically 1 to 5 nm thick and exist in a form of nanoflakes. The flakes are typically not flat, but with a twisted shape similar to flower petals. The diameter of the flakes is preferably in the range of a few micrometers and they typically have interplanar distances similar to that of graphite.

Generally, the nanoscale flakes or platelets preferably have an average length in the range of 5-50 microns, and preferably in the range of 10-40 microns, and more preferably in the range of 15-35 microns, and most preferably in the range of 20-30 microns.

The nanoscale flakes or platelets preferably have an average thickness in the range of 0.01-20 nm, and preferably in the range of 0.1-15 nm, and more preferably in the range of 0.5-10 nm, and most preferably in the range of 1-5 nm.

The nanoscale flakes or platelets preferably have an average width in the range of 1-30 microns, and preferably in the range of 1-20 microns, and more preferably in the range of 2-15 microns, and most preferably in the range of 3-15 microns.

The nanoscale flakes or platelets are preferably made of graphene or graphite. Preferably, the flakes/platelets are of essentially pure graphene and/or graphite. However, it is also feasible to use doped materials. For example, the graphene may be doped with boron, nitrogen or the like, or modified in other, per se known ways. However, other 2D materials may also be used, such as graphyne, germanene, silicone, phosphorene, boron nitride, etc, may also be used. All the flakes are preferably of one material. However, combinations of flakes made of two or more materials are also feasible.

The filler material may also comprise additional constituents, in addition to the nanoscale flakes or platelets. However, preferably the filler material comprises essentially only nanoscale flakes or platelets, and preferably consists of nanoscale flakes or platelets.

In a preferred embodiment, the filler material comprises at least 90 wt-% carbon, and preferably at least 95 wt-% carbon, and more preferably at least 99 wt-% carbon, and most preferably at least 99.9 wt-% carbon.

By the alignment of the nanoscale flakes or platelets obtained in the processing, and by the subsequent etching/ablating of the surface, the nanoscale flakes or platelets extend out from the surface. Thus, the platelets/flakes hereby comprise one free end, at a certain distance from the polymer matrix, and one end embedded in the polymer matrix. The flakes/platelets are hereby fixed to the surface by the polymer matrix material.

The flakes/platelets are preferably arranged with the longitudinal direction being essentially perpendicular to the surface. However, the orientation of the flakes/platelets may alternatively be slightly bent or tilted. Preferably, the longitudinal direction of the flakes/platelets are at an angle relative to the surface within the range 60-120 degrees, and preferably within the range 70-110 degrees, and more preferably within the range 80-100 degrees, and most preferably within the range 85-95 degrees.

Nanocomposites based on polymers, and preferably thermoplastic polymers, as a matrix have the advantage of being processable using mass production techniques such as extrusion and injection molding, techniques widely used in the biomedical devices industry. Other advantages include chemical resistance and sterilization compatibility, and, due to the fact that thermoplastic polymers can be re-molten and re-shaped, the possibility of recycling.

The polymer matrix is preferably a thermoplastic polymer, such as polycarbonates (PC), polyvinyl chloride (PVC), polyurethanes, copolymers comprising ethylene, such as polyethylene co-acetate, and polyolefin based elastomers, such as polypropylene (PP), styrene-ethylene-butylene-styrene (SEBS) and polyethylene (PE). Preferably the thermoplastic polymer is as low density polyethylene (LDPE), low-density polybutylene (LDBE), and/or polypropylene (PP).

In a preferred embodiment, the polymer matrix is formed by LDPE. LDPE is a long chain branched polyethylene thermoplastic. LDPE has excellent resistance to chemicals, except for halogenated hydrocarbons, and is unreactive at room temperature unless in the presence of a strong oxidizing agent. LDPE can withstand temperatures of up to 90 °C. It has been shown that the shear flow inside an extrusion die can be utilized to induce strong orientation and uniform distribution in the flow direction. This has been obtained for shear rates as low as 0.02 1/s and low extrusion temperatures, only slightly above the melting point of LDPE.

The inventors have found that controlled orientation of nanoscale flakes/platelets, such as graphite nanoplatelets (GNP), in bulk polymer nanocomposites can be used to produce a very good antimicrobial effect, and in a very controllable and cost-efficient way.

The processing of the mixture, providing the orientation and alignment of the nanoscale flakes or platelets, is preferably at least one of an extrusion process and an injection molding process. In one embodiment the processing is an extrusion process.

The processing is preferably arranged to provide a homogenous mixture of polymer matrix material and filler material.

The mixture of filler material and polymer matrix material preferably comprises 3 wt-% or more of filler material, and preferably 5 wt-% or more, and more preferably 10 wt-% or more, and most preferably 15 wt-% or more. Preferably, the amount of filler material is in the range of 3-40 wt-%, and preferably in the range of 5-30 wt-%, and more preferably in the range of 6-25 wt-%, and more preferably in the range of 7.5-20 wt-%, and most preferably in the range of 10-15 wt-%. However, as long as an adequate orientation of the flakes/platelets is achieved, the upper limit is of less importance, and greater amounts of filler may also be used.

The step of providing a surface of the processed mixture preferably comprises cutting of the processed material, and preferably in an essentially transversal or longitudinal direction in relation to a flow direction through the die.

The etching/ablating of the surface is preferably made so that the nanoscale flakes or platelets, on an average, extends from the polymer matrix material of the surface by a length in the range of 0.5-30 microns, and preferably in the range of 1-25 microns, and more preferably in the range of 2-20 microns, and most preferably in the range of 3-15 microns.

Preferably, the etching/ablating of the surface is made so that the nanoscale flakes or platelets, in an average, extends from the polymer matrix material of the surface by a length being at least 30% of the length of the flakes/platelets, and preferably at least 40%, and more preferably at least 50%.

The distance between any adjacent nanoscale flakes may be less than 10 µm, and preferably less than 5 µm, and most preferably less than 1 µm. Since this is in correspondence with the size of prokaryotic cells, the killing effect hereby becomes more efficient.

The distance between any adjacent nanoscale flakes may be more than 0.01 µm, and preferably more than 0.05 µm, and most preferably more than 0.1 µm. Since the killing effect may be reduced in case the flakes are arranged too densely, a less dense arrangement may often be preferred.

In experiments, it has been shown that e.g. GNP-LDPE composites can be extruded in controlled conditions in order to obtain orientation of GNP flakes within the polymer matrix (LDPE). After extrusion, nanocomposite samples were etched/ablated to wash away unwanted polymeric traces and to expose the edges of oriented GNP flakes. This resulted in concentration-dependent distribution of GNP flakes on the surface of the etched/ablated extruded nanocomposite samples. The antimicrobial activity of GNP-LDPE composites was evaluated against opportunistic bacterial pathogens, *Eescherichia coli* and *Staphylococcus epidermidis.* The results from the antimicrobial assessment shows that GNP nanoflakes on the extruded GNP-LDPE composite interact with the bacterial cell membrane physically, thereby damaging the cells. The observed bactericidal activity was dependent on the density of oriented GNP flakes on the composite surface. Furthermore, samples with higher density of GNP flakes (15% and above) prevented bacterial colonization. These results show that GNP-LDPE composites can be used e.g. in polymer based biomedical devices with bactericidal activity to mitigate device associated infections. These biomedical devices would be competitive on the global market due to the low cost of source materials and the industrially scalable method of manufacturing, e.g. of GNP-LDPE composites.

The obtained bactericidal effect is similar to the one discussed in US 2018/320002, but surprisingly even further improved.

The antibacterial effect is mechanical. Without wanting to be bound by any theory, it is assumed that the sharp edges of the nanoscale flakes are able to come in contact and penetrate a layer of membrane lipids by forming strong van der Waals attractions. This leads to the membrane rupture, because the lipid hydrophobic tails of the membrane lipids get spread out on the nanoscale flake surface, attracted by strong hydrophobic interactions.

The reason that eukaryotic cells are not killed in the same way is assumed, again without wanting to be bound by any theory, to be due to the larger size of eukaryotic cells compared to prokaryotic cells, and/or the thicker walls/membranes of eukaryotic cells. Eukaryotic cells are generally about 10 times as big as prokaryotic cells. Prokaryotic cells are generally of a size within 1-10 µm. Most bacteria are smaller than 1 µm in at least one dimension, but can be elongate and somewhat longer, up to 10 µm in a length direction. The thickness of the cell membrane varies somewhat between various cells, but is typically about 10 nm for Gram-negative bacteria, but can be 20-80 nm for Gram-positive bacteria, if including the rather thick layer of peptidoglycan. Eukaryotic cells are generally of a size within 10-100 µm.

Graphene and thin graphite, such as e.g. nano-graphite, are, similar to many other 2D materials, generally believed to be hydrophobic, in comparison to e.g. graphene oxide and graphite oxide, which are less hydrophobic or even hydrophilic. It is assumed that this hydrophobic nature of the nanoscale flakes has a beneficial effect on the killing of the bacteria.

Since the killing of the bacteria is mechanical, and attached to the surface, many of the disadvantages of known ways to provide antibacterial effects, such as the use of antibiotics, are prevented. There is for example no risk of bacteria resistance and harmful side effects.

Since the nanoscale flakes/platelets are efficient to kill and inhibit growth of prokaryotic cells, i.e. bacteria and the like, and do not kill eukaryotic cells, the coating/surface can also be used in direct contact with skin and tissue of humans and animals, without any negative or detrimental effects.

The thickness of at least some of the nanoscale flakes/platelets, and preferably all the nanoscale flakes/platelets, may be tapering towards the free end, opposite the edge side embedded in the polymer matrix. Additionally or alternatively, the width of at least some of the nanoscale flakes, and preferably all the nanoscale flakes, may be tapering towards the free end, opposite the attached edge side. Hereby, the attachment to the surface may be stronger, and the killing of bacteria becomes more efficient.

According to another aspect of the invention, there is provided an article having an antibacterial surface, wherein the article comprises a substrate comprising a polymer matrix and a filler material comprising nanoscale flakes or platelets, wherein said article comprises an antibacterial surface having the nanoscale flakes or platelets essentially aligned to each other and extending out from said surface with a length in the range of 0.5-30 microns. Preferably, the nanoscale flakes or platelets extend out from the surface by a length in the range of 1-25 microns, and more preferably in the range of 2-20 microns, and most preferably in the range of 3-15 microns.

The article may be provided as a coating, and preferably as a coating on a medical device, such as a catheter, and preferably a urinary catheter.

Hereby, similar properties and advantages as discussed above in relation to the other aspects of the invention are obtainable.

These and other aspects of the inventive concept will be apparent from and elicited with reference to the embodiments described hereinafter.

The antibacterial coating/surface of the present invention is useful for many different types of devices.

In one embodiment, the device may be a medical device, such as a catheter, medical tube, irrigation device or implant. In particular, the antibacterial coating/surface is well suited for medical devices which are to be used many times, such as irrigation devices, and/or for a long time, such as indwelling urinary catheters, so-called Foley catheters. In catheters, the antibacterial coating/surface may be arranged either on an internal surface, inside the lumen, and/or on an external surface. In an irrigation device, the antibacterial coating/surface may be arranged in the probe, internally and/or externally, but may additionally or alternatively be provided internally in tubes of the device, such as tubes leading irrigation liquid to the probe for irrigation. In implants, the coating/surface may e.g. be arranged close to surrounding tissue, thereby improving and speeding up the tissue healing after having inserted the implant, and reducing the need for antibiotic drugs. The implant, may e.g. be a dental implant, but may also be other types of implants.

In one embodiment, the device may form an internal lumen, and wherein said antibacterial coating/surface is arranged on an internal surface of the substrate facing said lumen. Thus, the device may e.g. be a tube or having a generally tubular shape. For example, the coating/surface may be arranged in tubes or pipes for transporting water, thereby reducing the amount of bacteria in the water, tubes and pipes for transporting beverages, medical tubing, catheters, and the like.

The antibacterial surface is also useable in many other types of applications, where there is a need to reduce the presence and colonialization of bacteria. For example, the antibacterial surface is of great advantage in the food industry, and e.g. in packages and the like for food.

The antibacterial surface may also be used on ship and boat hulls, to reduce growth and to reduce the need for anti-fouling paints.

Further, the device may be, or forms part of, a cell growing or cell culture equipment. Hereby, the use of antibiotic drugs, which is today commonplace, could be omitted or at least strongly reduced. Since the antibacterial coating/surface kills prokaryotic cells but without harm to eukaryotic cells, the coating/surface efficiently kills bacteria without affecting the cultured eukaryotic cells. Hereby, cells, such as skin cells, stem cells, etc, can be grown and cultured more efficiently, and with reduced negative side effects, both on the cells themselves and on the environment.

### Brief description of the drawings

By way of example embodiments of the invention will now be described with reference to the accompanying drawings in which:
Figs. 1a and 1b are flow charts schematically illustrating exemplary embodiments of a production of an antibacterial surface, in accordance with embodiments of the present invention;
Figs. 2a and 2b are cross-sectional views in a plane parallel to an extrusion direction, schematically illustrating orientation and alignment of nanoscale flakes or platelets during extrusion in the methods of Figs. 1a and 1b;
Figs. 3a and 3b are cross-sectional views in a plane perpendicular to an extrusion direction, schematically illustrating orientation and alignment of nanoscale flakes or platelets during extrusion in the methods of Figs. 1a and 1b;
Figs. 4a and 4b are cross-sectional views schematically illustrating etching/ablating of a surface to expose the flakes/platelets in the methods of Figs. 1a and 1b;
Fig. 5 is a schematic illustration of an extrusion apparatus useable in the methods of Figs. 1a and 1b;
Figs. 6-8 are schematic illustrations of how orientation and alignment of the flakes/platelets is obtained in methods according to the present invention;
Fig. 9 are SEM pictures illustrating different antibacterial surfaces in accordance with embodiments of the present invention;
Fig. 10 are diagram showing the experimentally determined antibacterial activity of the surfaces of Fig. 9;
Fig. 11 are fluorescence microscope pictures illustrating live/dead bacterial viability of the surfaces of Fig. 9;
Fig. 12 are SEM pictures showing bacteria in contact with the surfaces of Fig. 9; and
Fig. 13 is a schematic illustration of the antibacterial activity of antibacterial surfaces in accordance with embodiments of the present invention.

### Description of preferred embodiments

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations of the invention.

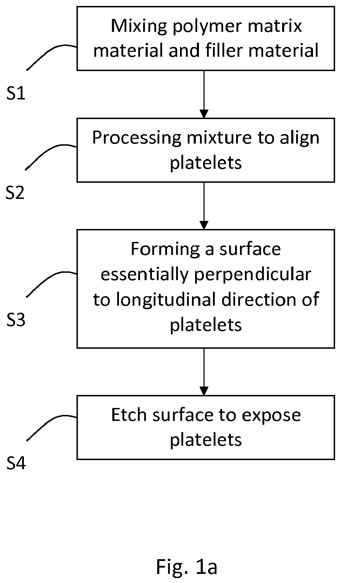

In a production method in accordance with a first embodiment, as illustrated schematically in the flow chart of Fig. 1a, the method comprising mixing of a polymer matrix material and a filler material, wherein the filler material comprises nanoscale flakes or platelets, step S1. Thereafter, in step S2, the mixture is processed to align the platelets within the mixture. This can e.g. be made by extrusion of the mixture, but other types of processing, such as injection molding, can alternatively be used.

Then, in a step S3, a surface is formed in the extruded part, so that the surface extends essentially perpendicularly to the longitudinal direction of the aligned platelets. This forming may e.g. be made by cutting the extruded part in a certain direction, such as in a transversal or longitudinal direction in relation to the extrusion direction.

Finally, in step S4, the surface is etched or ablated, thereby removing the uppermost part of the polymer matrix material, so that the platelets previously being embedded close to the surface now extend out from the surface.

As discussed in the foregoing, different polymer matrix materials and filler materials may be used. In a specific embodiment, LDPE is used as a polymer matrix material, and GNP is used as filler material.

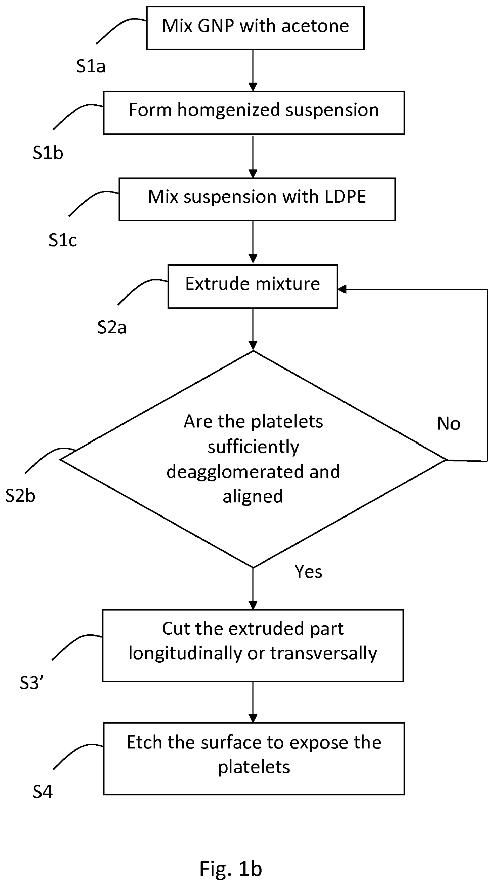

A method for producing an antibacterial surface using LDPE and GNP is illustrated in Fig. 1b.

Here, in step S1a, the GNP is initially mixed in a liquid, such as acetone, to deagglomerate the platelets from each other. However, depending on the quality etc of the filler material, such initial deagglomeration may be unnecessary, and may then be omitted.

In step S1b, the GNP and acetone suspension may be homogenized and further deagglomerated in an additional mixing step. However, this step is also optional, and may in many cases be omitted.

Exfoliation, e.g. in a high shear mixer, may also be used in order to reduce the thickness of the nanoplatelets by separation of their layers.

The suspension is then, in step S1c, mixed with LDPE, preferably provided in the form of powder. The LDPE and GNP are hereby preferably mixed and stirred to form a homogeneous mixture, and until the acetone has evaporated. However, other ways of forming the mixture are feasible, and in certain embodiments, mixing and deagglomeration can be made directly in the subsequent processing, such as in an extrusion process.

The mixture is then, step S2a, feed into an extruder, whereby the platelets of the filler material will be aligned so that their longitudinal directions extend generally in the extrusion direction. Depending on the materials used, how much homogenization and deagglomeration that was made prior to extrusion, how much alignment that is required, etc, it may be necessary to run the material through the extruder more than one time, such as 2-10 times, and in particular 2-5 times. As a rule of thumb, the platelets will be more aligned and better dispersed with every additional round of extrusion.

When a sufficient degree of deagglomeration and alignment has been obtained, step S2b, which may be determined by evaluation of the produced material, or based on empirical knowledge from previous batches, the extruded material is removed. The material is then, in step S3', cut in a direction perpendicular to the length direction of the aligned platelets within the material. The cutting may e.g. occur longitudinally or transversally, as seen in the extrusion direction.

Finally, in step S4, the surface is etched/ablated, thereby to expose the outermost platelets, but while still being partly embedded within the polymer matrix material.

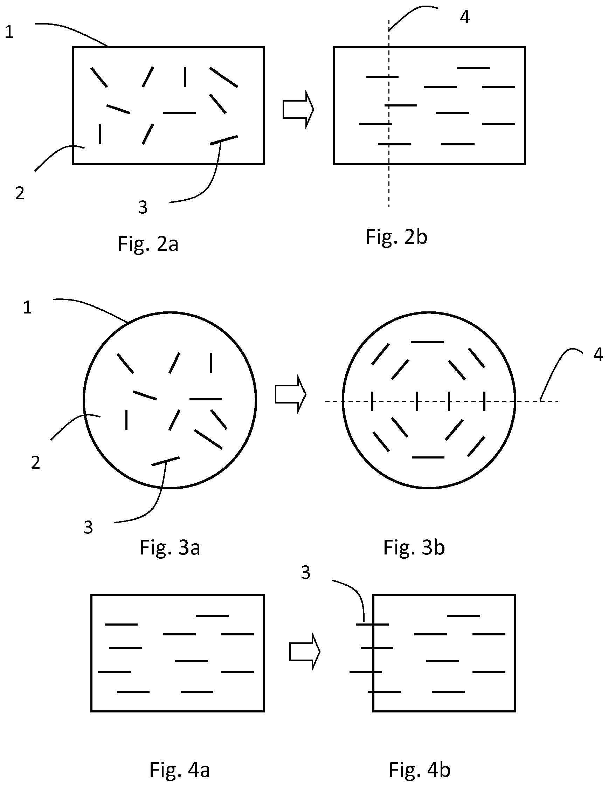

The process is further illustrated schematically in Figs. 2-4.

In Fig. 2a, a schematic cross-sectional view in the longitudinal direction of the material 1 is illustrated. Prior to extrusion, or after an insufficient number of extrusion rounds/loops, the platelets 3 are embedded in the polymer matrix material 2, but arranged in random, non-aligned orientations. After sufficient number of rounds in the extruder, such as 1-3 rounds, the platelets will be aligned in the extrusion direction, as schematically illustrated in Fig. 2b.

This material may then be cut in a transversal direction, illustrated with dashed line 4, to form the antibacterial surface.

In addition to being aligned in their length directions, the platelets will also be aligned in their width directions. This alignment occurs around concentric circles around the center of the extruded part, so that each major side of the platelets is facing either towards the center or away from the center. This is schematically illustrated in Figs. 3a and 3b.

In Fig. 3a, a schematic cross-sectional view in the circumferential direction of the material 1 is illustrated. Prior to extrusion, or after an insufficient number of extrusion rounds, the platelets 3 are embedded in the polymer matrix material 2, but arranged in random, non-aligned orientations. After sufficient number of rounds in the extruder, such as 1-3 rounds, the platelets will be aligned in concentric circles around the center, as schematically illustrated in Fig. 3b.

This material may then be cut in a longitudinal direction, illustrated with dashed line 4, to form the antibacterial surface.

The surface is etched/ablated to remove loose platelets, and to lower the surface of the polymer matrix material. This is illustrated schematically in Figs. 4a and 4b, where Fig. 4a illustrate the material prior to etching/ablating, and where Fig. 4b illustrate the same material after etching/ablating. After etching/ablating, the platelets 3 will extend out from the surface and from the polymer matrix material, but still be embedded in the polymer matrix material at the other ends.

The removal of the polymer matrix material at the surface can be obtained by etching, such as chemical wet etching, e.g. by use of strong acids. However, other methods may also be used, such as plasma etching/ablation, laser etching/ablation, etc.

By the above-discussed processing, the platelets are orientated in the desired way within the produced material, and are further sufficiently deagglomerated and dispersed.

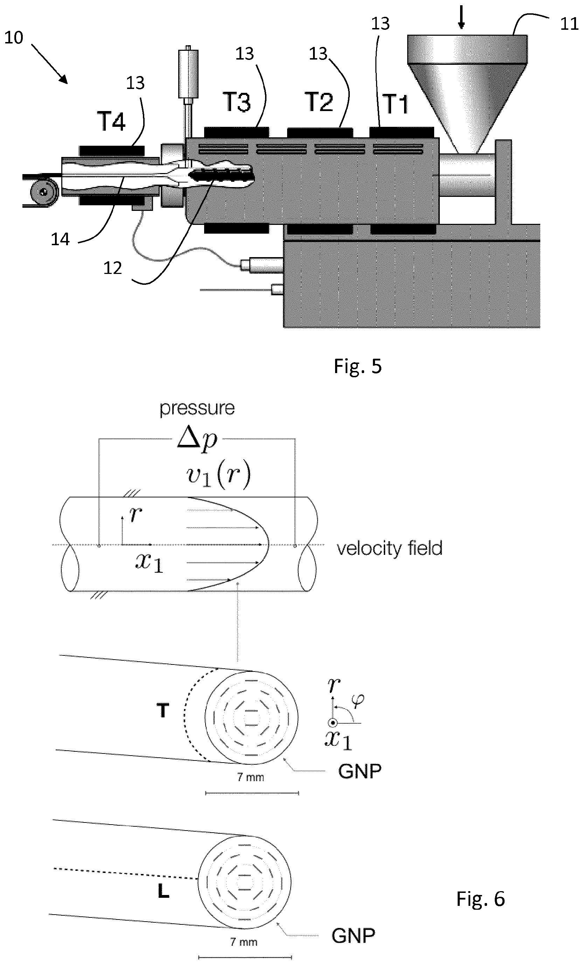

Extrusion processing is per se well known, and need not be discussed in detail. An exemplary extruder is illustrated in Fig. 5. The extruder 10 may have an inlet, a so-called hopper 11, an extruder screw 12, one or several heaters 13, which may provide zones T1-T4 with controllable temperatures, and a nozzle or die 14.

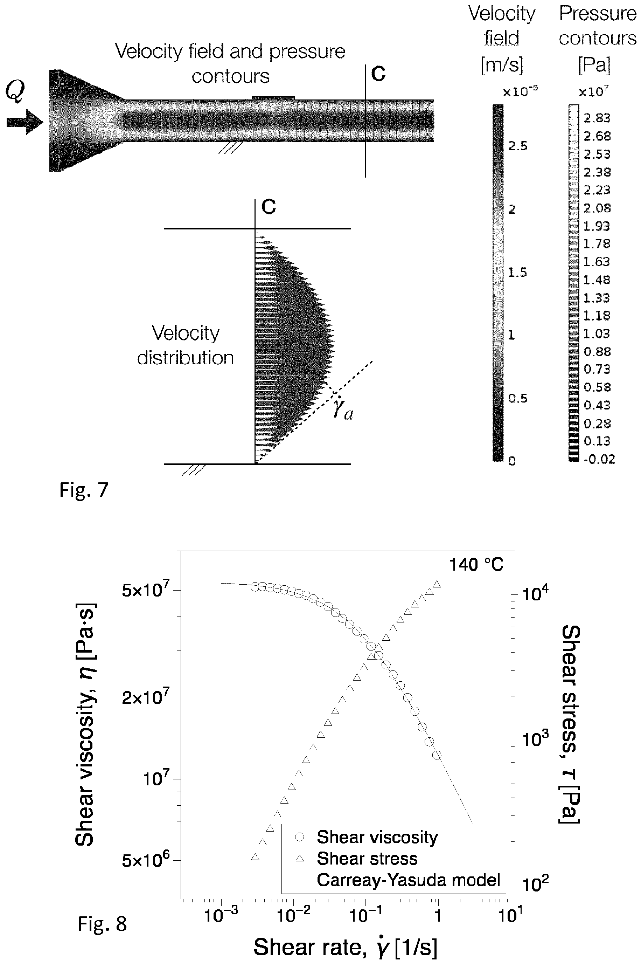

The polymer matrix material is heated to a temperature above the melting temperature during extrusion, and the velocity gradient inside the die will form a parabolic velocity profile, as illustrated in Figs. 6 and 7. Deviations from this will occur if the local shear rates fall within the shear-thinning region of the viscosity function. Therein, the nanoplatelets will align with the principal axis in the extrusion flow direction and the secondary axis along concentric iso-velocity cylindrical planes, as illustrated in Fig. 6, schematically illustrating the flow induced morphology. Deviations from the expected orientation could occur in the presence of low velocity gradients, e.g. in the center of the flow channel especially for non-Newtonian fluids where the velocity profile is flattened in the center.

In addition to the velocity gradient requirements for orientation, the induced stresses - shear stresses in the case of steady state flow inside the die - should preferably be sufficiently high to ensure a good breaking of filler agglomerates.

It has been shown experimentally given the same kinematic conditions, a polymer matrix material with higher viscosity resulted in the most consistent de-agglomeration and subsequent orientation and was selected for the study.

Fig. 8 presents the viscosity function and flow curve for LDPE. The screw speed was thus chosen such that the apparent shear rate (***γ̇***) inside the die would fall as close as possible to the Newtonian plateau of the polymer matrix. Numerical solution of the Poiseuille flow inside the die using the viscosity function in Fig. 8 for a screw speed of 30 rpm is shown in Fig. 7.

Generally, it is preferred to use a polymer matrix material having relatively high viscosity, since it facilitates the process of orienting and aligning the flakes/platelets. However, materials having lower viscosity may also be used, and then preferably with use of higher pressure in the extrusion process.

Generally, the shear stress is a function of viscosity and shear rate, and it is believed that a high shear stress is beneficial, since it improves de-agglomeration of the flakes/platelets, and that a high shear rate is beneficial, since it improves orientation and alignment of the flakes/platelets.

Generally, it is also preferred to provide the processing, such as the extrusion, at relatively low temperatures, above but relatively close to the melting temperature of the polymer matrix material. Preferably, the processing temperature(s) is/are less than 100 degrees above the melting temperature of the polymer matrix material, and preferably less than 50 degrees above said melting temperature.

The die may have any shape. In one embodiment, the die has a circular opening, producing an extrudate in the form of a cylindrical rod. However, other shapes, are also feasible, such as using a rectangular die opening, to produce an extrudate in the form of a plate or the like.

In some applications, the extrusion or injection molding can be used to form a device, such as a medical device, whereby the antibacterial surface may be provided directly on a surface of the produced device. However, alternatively, the antibacterial surface can be produced separately, such as in the form of sliced stripes, ribbons or the like, which may then be attached to a surface of a device like a coating.

In case the antibacterial surface is produced in the forms of stripes or ribbons, the rest of the material may be recycled into the process, and again used for extrusion or injection molding.

### Examples and experiments

A practical example of preparation of GNP-LDPE nanocomposites will now be discussed.

The GNP-LDPE composites were obtained by the method illustrated in Fig. 1b. For this purpose, low density polyethylene (Borealis AB, Stenungsund, Sweden) was used. Characteristics of the LDPE, based on Gel Permeation Chromatography and Differential Scanning Calorimetry is presented in Table 1 below.

GNP grade M25 was obtained from XG Sciences (Lansing, USA) for use as a filler. The properties of GNP as stated by the manufacturer are presented in Table 1 below, extracted from product data sheet.

**Table 1. Materials parameters**

| GNP | | LDPE | |
|---|---|---|---|
| *d_{avg}* [µm] | 25 | *M_{w}* [kg/mol] | 92 |
| *A* [m²] | 7.5 | *M_{w}*/*Mₙ* | 7.6 |
| ρ [g/cm³] | 2.1 | *Tₘ*/*T_{c}* [°C] | 111/94 |

The LDPE was cryogenically ground into powder form with particle size ca. 0.5 mm by using a high rotor mill. GNP M25 powder was mixed with acetone and homogenized by means of sonication for 3 h (90 W).

The well dispersed and homogenized suspension of GNP with acetone was stirred for 20 min by means of rotor-stator mixer Ultra-turrax T 25 IKA at 15 000 rpm in order to get rid of agglomerates in the suspension. Thereafter, LDPE in the powder form was mixed with the GNP-acetone suspension using an overhead stirrer rotating for 40 min at 500 rpm until full evaporation of acetone has been obtained. This process was followed by drying the obtained master batches of composites with various concentration of GNPs at 60 °C for 24 h.

For experimental verification, different amount of filler material was used in the batches. Specifically, batches were produced having the filler concentration as follows: 5, 10, 15 and 20 wt-%.

The batch materials were then extruded. The extrusion process of GNP-LDPE nanocomposites was carried out using a circular die. LDPE-GNP master batches were extruded using a Brabender 19/25 D single-screw extruder (Duisburg, Germany) with a compression screw having diameter D = 19 mm and screw length of 25 × 19, and compression ratio 2:1. The extruder was equipped with Terwin 2000 series (model 2076) melt pressure sensors with maximum pressure of 700 bar for inline monitoring. The used temperatures, from the compaction zone, melting and metering zones to the extruder's die, were as follows 115, 130, 130 and 140 °C. The used screw speed of 30 rpm was maintained during the whole process.

The extrusion process was repeated three times. The first two extrusions were treated as a melt compounding of the LDPE-GNP master batches to obtain proper dispersion of the nanofiller within the polymer matrix. Finally, the master batch was extruded into cylindrical extrudates with a diameter of 7 mm. Specimens for antibacterial analysis were collected after the inline pressure reached steady state. Specimens were made with the filler concentration as follows: 5, 10, 15 and 20 wt-%.

The extruded cylindrical samples were cut in transverse directions T, in a plane perpendicularly to the flow direction, as illustrated in Fig. 2b and Fig. 6, and longitudinally directions L, in a plane parallel to the flow direction containing the axis of the cylinder, as illustrated in Fig. 3b and Fig. 6. The cutting was made with a low speed saw with diamond blade.

Afterward, the surfaces of the samples were etched or ablated in order to expose the GNP nanoflakes. For this purpose an etchant comprising sulfuric acid, ortho-phosphoric acid and water with 1 wt. % of potassium permanganate was used for 30 h. After the etching/ablating all the samples were carefully cleaned with the mixture of sulfuric acid and deionized water followed by hydrogen peroxide and rinsed twice with deionized water.

The thus produced samples with antibacterial surfaces were then tested to evaluate the antibacterial activity. The antimicrobial response of extruded and etched/ablated samples of different concentration of GNP-LDPE composites was evaluated by using colony counting method, scanning electron microscopic observation and live/dead viability staining. The antimicrobial activity of extruded samples was tested against the E. coli and S. epidermidis.

Overnight growth of respective bacterial cultures was diluted to obtain final inoculum of 2-5 × 106 CFU/ml and seeded on the surface of LDPE (Control) as well as on the LDPE nanocomposite samples containing various concentration of GNP. Bacterial inoculum loaded samples were incubated for 24 h at 37 °C to grow the respective biofilms on the surfaces. After the 24 h, biofilms were rinsed twice with sterile water and collected in 5 ml of 0.89% of sodium chloride. Biofilms were dispersed and homogenized by sonication (30 sec). The homogenized biofilm suspension (100 µl) was serially diluted and plated on agar plate, incubated at 37 °C to count colonies.

To examine the interaction between GNP nanoflakes and the bacterial cells, biofilms were observed with a scanning electron microscope. The biofilms grown on LDPE surface as well as surfaces with various concentration of GNP were fixed with 3% of glutaraldehyde for 2 h. The fixed biofilms were dehydrated by using graded series of ethanol concentrations (40, 50, 60, 70, 80 & 90%) for 15 min each and with absolute ethanol for 20 min. All the dehydrated biofilms were dried overnight at room temperature and sputter coated with thin layer of gold (5 nm) before SEM imaging. SEM imaging was performed with Supra 55 VP (Carl Zeiss AG, Jena, Germany). To further confirm the bactericidal activity of LDPE-GNP composites, biofilms were stained with LIVE/DEAD® BacLight Bacteria Viability stains kit L7012 (Invitrogen, Molecular Probes, Inc. Eugene, OR, USA). The kit contains the green-fluorescent nucleic acid stain SYTO® and the red-fluorescent nucleic acid stain propidium iodide (PI). The green-fluorescent dye (Syto) crosses all bacterial membranes and binds to the DNA of bacterial cells and containing second dye, red-fluorescent PI that only crosses damaged bacterial membranes (dead bacteria). After the staining with mixture of Syto and PI for 20 min, Fluorescence microscopic imaging of the biofilms was performed using a Zeiss fluorescence microscope (Axio Imager.Z2m Carl Zeiss, Jena, Germany).

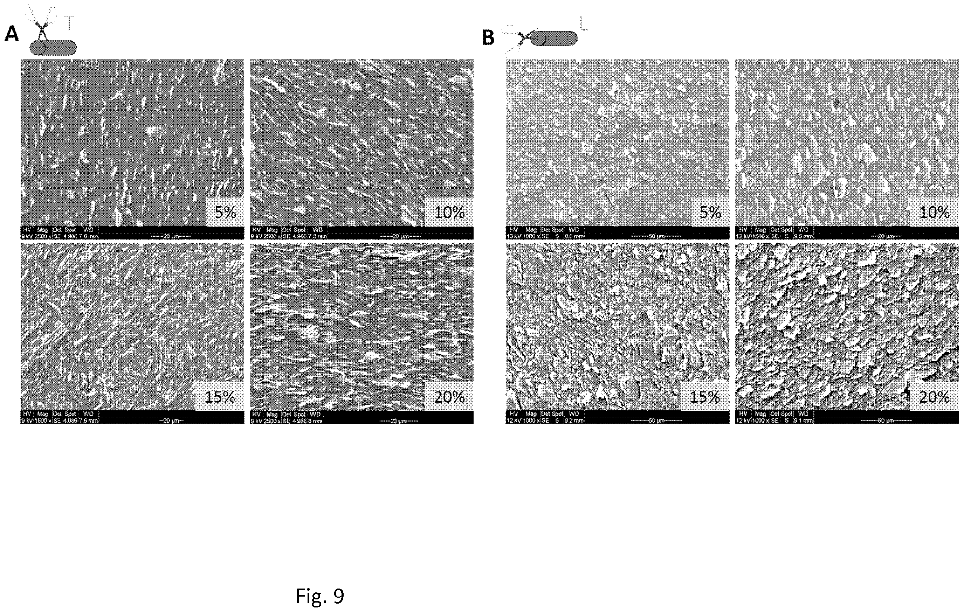

From SEM pictures one can see that, GNP nanoflakes are oriented in the polymer flow direction. The SEM pictures of group A, to the left in Fig. 9, show samples cut in a transversal direction at four different GNP concentrations, and the SEM pictures of group B, to the right in Fig. 9, show samples cut in a longitudinal direction, at the four different GNP concentrations. All pictures clearly show the vertical orientation of the GNP nanoflakes.

The antimicrobial activity of these samples was tested against pathogenic bacteria commonly causing infections: *E. coli* and *S. epidermidis.* To assess bactericidal activity of vertically oriented GNP nanoflakes against both Gram-positive and Gram-negative bacteria, *E. coli* was used as a model organism for Gram-negative bacteria and *S. epidermidis* was used as a model of Gram-positive bacteria.

There are a variety of existing strategies for the evaluation of antimicrobial activity. Here we have grown the bacterial cultures on the composite surfaces cut in T (transverse) and L (longitudinal) orientation with respect to direction of extrusion prior to etching/ablating. The reason for the samples being cut in these two ways, has to do with orientation with respect to direction of extrusion and edge-effects. Colony forming units (CFUs) of adherent bacteria were first determined by plating method.

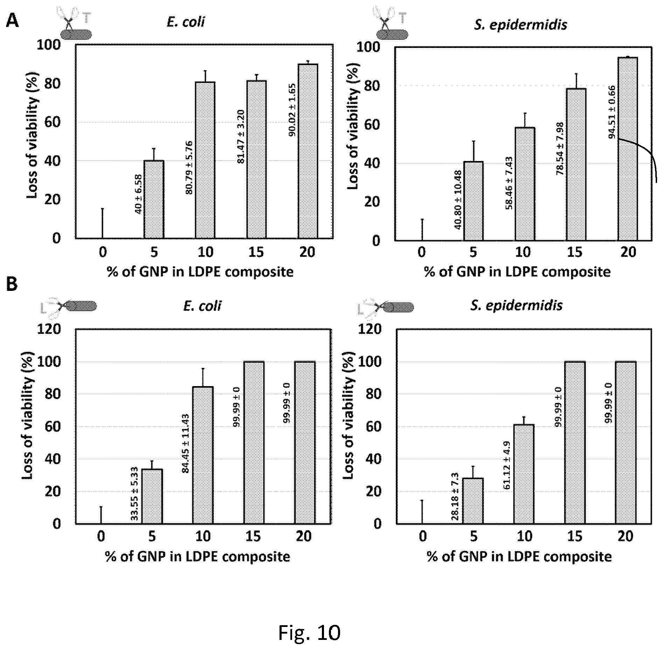

Significant loss in viability of both *E. coli* and *S. epidermidis* was observed on all composite surfaces with GNP regardless of cutting direction, as shown in the diagrams of Fig. 10. The loss in viability was dependent on the concentration of GNP in the GNP-LDPE composites. Even the 5 wt-% of GNP-LDPE composites showed significant bactericidal impact. With T cut samples, the loss of viability was 40 ± 6.5% and 40.8 ± 10.5% against *E. coli* and *S. epidermidis,* respectively. In the L cut samples, the loss of viability was 33.55 ± 6.5% and 28.18 ± 7.3% against *E. coli* and *S. epidermidis* respectively. The loss in viability of *E. coli* jumped to 80.8 ± 5.8% and 84.45 ± 11.43% with T and L sliced 10 wt-% of GNP-LDPE composite, and for the same samples, loss in viability of *S. epidermidis* raised to 58.5 ± 7.4% and 61.12 ± 4.9%, respectively, with T and L sliced sample. This considerable difference in the bactericidal activity of 10% GNP-LDPE composites against *E. coli* and *S. epidermidis* might be due the shape of the bacterial cells rather than being gram positive and gram negative bacteria. In the 10 wt-% of GNP-LDPE composites, though the density of oriented GNP nanoflakes appears well distributed and homogenous, there are gaps in between nanoflakes with enough space for circular *S. epidermidis* cells to attach without interference. Whereas *E. coli* cells, being rod shaped (with 2 µm length), are more likely to get in contact with the GNP nanoflakes.

The loss in viability of *S. epidermidis* with 15 wt-% of GNP-LDPE was raised to 78.5 ± 8.0% and 99.99 ± 0% with T and L sliced composite sample, whereas bactericidal effect against *E. coli* remain similar (81.47 ± 3.20%) as for 10 wt-% of GNP-LDPE with T sliced sample. In the case of L sliced sample, loss in viability of *E. coli* raised to 99.99 ± 0%.

Moreover, with the 20 wt-% of GNP-LDPE, the loss in viability went up to 90.0 ± 1.7% and 94.5 ± 0.7% of *E. coli* and *S. epidermidis,* respectively, with T sliced samples. On the other hand, 99.99% of loss in viability of both *E. coli* and *S*. *epidermidis* was observed with L sliced composite samples.

The similar loss in viability % of *E. coli* (gram negative bacteria) and *S*. *epidermidis* (gram positive bacteria) observed with the 20 wt.% of GNP-LDPE indicates that antimicrobial efficacy of the material is solely dependent on filler content and geometry of graphite nanoplatelets flakes on the surface, regardless to the different group or type of bacteria. The anti-biofouling activity of the composite material seen with CFUs counting method might be due to bactericidal activity or inhibitory effect on bacterial adhesion, or due to both bactericidal and anti-adhesion activity.

Moreover, L sliced samples have shown more pronounced antibacterial effect than T sliced. This behavior is probably associated with the fact that the L sliced samples had slightly greater area than T sliced samples, which resulted in higher number of bacteria in contact with GNP flakes for L sliced samples.

The wetting by the media was also more efficient for the L sliced samples than for the T sliced samples, which also can influence the loss in viability %.

From these measurements, the following may be concluded:
- Even with a low GNP concentration of 5 wt-%, a good antibacterial effect was obtained.
- At higher GNP concentrations, at 10 wt-% or above, a very good antibacterial effect was obtained.
- At GNP concentrations of 15 wt-%, extremely good antibacterial effects were obtained.
- The antibacterial effect was even slightly further improved when 20 wt-% GNP was used.

Furthermore, to confirm the inhibitory effect on bacterial adhesion and bactericidal effect of GNP-LDPE composites, biofilms formed on control as well as composite samples were stained with live/dead bacterial viability kit and examined under the fluorescence microscope. Using fluorescence probe to detect the viability of bacteria is another widely used method to evaluate antimicrobial activity. SYTO at the concentration of 6 µM is able to cross cytoplasmic membrane and interact with the nucleic acid to enhance the fluorescence giving green fluorescence intensity. Propidium iodide (PI) exhibit a fluorescence enhancement of up to 20- to 30-fold upon intercalation into double-stranded regions of DNA. PI is unable to cross cytoplasmic membrane of bacteria, and consequently generates no fluorescence enhancement in live bacterial cells. In the case of compromised bacterial cell membrane, the used PI interact with nucleic acid and enhance red fluorescence intensity. Hereby, the bacterial cells damaged by vertically oriented GNP flakes are stained red with the PI. The live bacteria in biofilms on control as wells as GNP-LDPE composites are stained green with the SYTO.

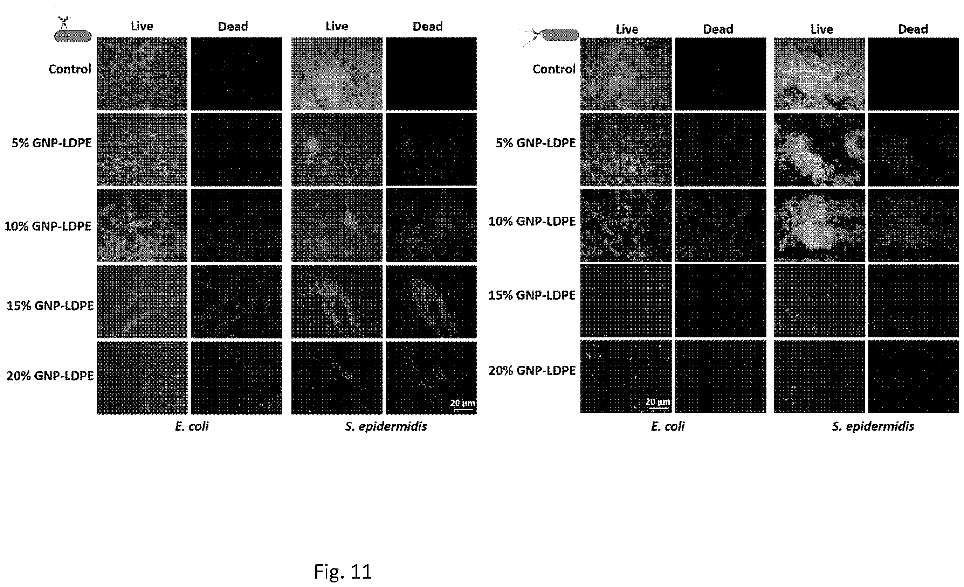

The results of this are shown in Fig. 11, where the pictures of the first four columns are related to T cut samples, and the last four columns to L cut samples. The first two columns of each group, i.e. columns 1-2 and 5-6, are related to *E. coli,* and the last two columns of each group, i.e. columns 3-4 and 7-8, are related to *S*. *epidermidis.* Columns 1, 3, 5 and 7 are showing the live bacteria, originally colored in green but here reproduced in greyscale, whereas columns 2, 4, 6 and 8 are showing the dead bacteria, originally colored in red but here reproduced in greyscale. The first row is directed to the control sample, without GNP, and the last four rows are directed to samples containing 5, 10, 15 and 20 wt-% of GNP.

As demonstrated in Fig. 11, overall density of biofilms of both *E. coli* and *S*. *epidermidis* were observed to be drastically decreased for the samples containing GNP in the composite compared to the control samples. The decrease in density of bacterial cells in higher concentration of GNP-LDPE composites directly correlates with the concentration of oriented GNP nanoflakes in the composite samples. Interestingly, very few bacterial cells were observed on the vertically sliced 15 and 20 wt. % of GNP-LDPE composite samples suggesting that higher concentration of GNP also plays a role in prevention of bacterial adhesion and as an effect prevention of colonization.

Further, the interaction between bacterial cells to vertically oriented GNP nanoflakes on GNP-LDPE composite was examined by using a SEM. Since very few bacterial cells were observed in 15 and 20 wt-% L cut samples, only T cut samples were used for SEM analysis.

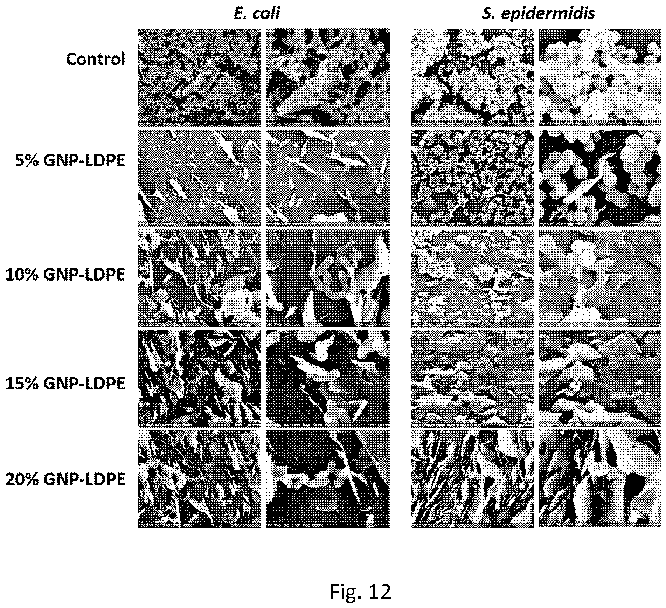

The SEM pictures are shown in Fig. 12. Here, the first and second columns are showing *E. coli,* where the first column has somewhat lower magnification and the second column somewhat higher magnification, and the third and fourth columns are showing *S. edipermidis,* where the third column has somewhat lower magnificaton and the fourth column has somewhat higher magnification. Row 1 shows pictures of the control sample, whereas rows 2-5 show pictures of the samples prepared with 5, 10, 15 and 20 wt-% of GNP.

Fig. 12 demonstrates the contact of bacterial cells to sharp edges of GNP and the damage of bacterial cells by piercing through the cell wall. In the case of lower concentration of GNP in GNP-LDPE composites, a small number of bacterial cells are observed to get in contact with the GNP nanoflakes. In the case of composite samples with higher concentration of GNP, the high amount of vertically oriented GNP nanoflakes enhances the possibility of bacterial cells getting in contact with nanoflakes and leading to the morphological disruption, thereby enhancing the loss in viability. The images from SEM examination follows the results obtained from the colony counting method indicating a higher percentage loss in viable cells in dependence on the concentration of GNP used and the amount of vertically oriented GNP nanoflakes on the GNP-LDPE composite surface.

The obtained results are consistent with the findings in US 2018/320002, suggesting contact killing or insertion mode of action by graphene. Furthermore, attachment of bacterial cells on the GNP/LDPE composite is observed to be significantly lower with the high concentration (15 wt. % and 20 wt. %) of GNP on composites materials. Prevention in the bacterial adhesion on the samples with higher concentration GNP suggests that the vertically oriented GNP nanoflakes inhibit the biofilm formation by at least two different mechanisms, including the prevention of colonization as well as physical damage of adhered bacterial cells.

### Conclusion and summary

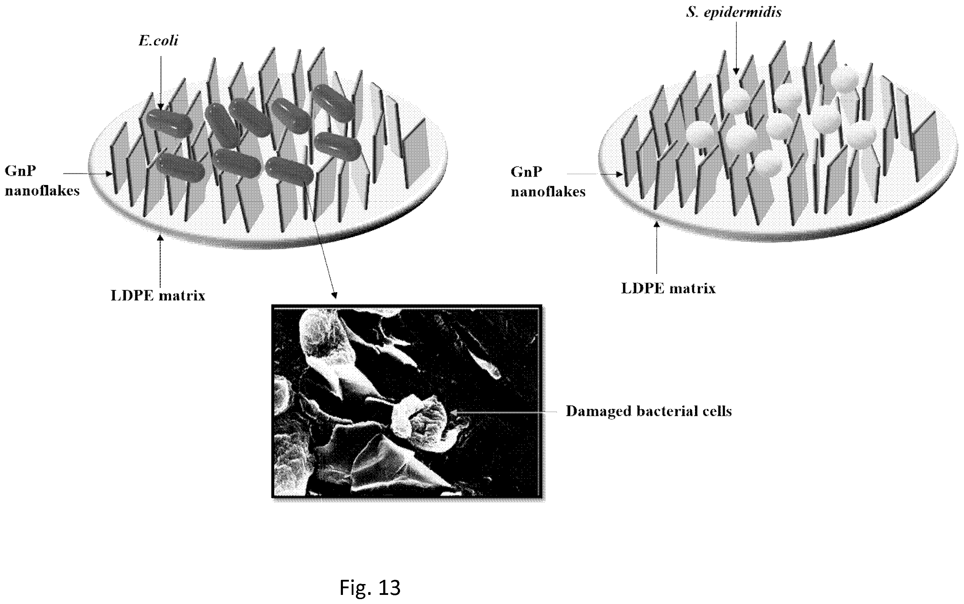

The results of the experiments clearly demonstrate that controlled orientation of GNP on the polymeric composite materials follows similar mechanism as other graphene derivatives to damage the microbial cells. The results from the antimicrobial assessment show that GNP nanoflakes on the extruded GNP-LDPE composite interact with the bacterial cell membrane physically thereby damaging the cells. This is illustrated schematically in Fig. 13.

The bactericidal activity is further dependent on the density of oriented GNP flakes on the composite surface, and it was found that particularly advantageous results were obtained when the amount of filler material was 10 wt-% or more. Furthermore, samples with higher concentration of GNP flakes was shown to have strong efficacy to inhibit bacterial colonization. The results show that GNP-LDPE composites can be used to develop e.g. polymer based biomedical devices with bactericidal activity that could prevent possible device associated infections. The production method is fast and cost-efficient, and can easily be scaled for industrial production.

The invention has now been discussed in relation to different embodiments. However, it should be appreciated by those versed in the art that several further alternatives are possible. For example, the antibacterial coating/surface may be used in many other medical devices, as well as in other applications. For example, the coating/surface may be used in other types of catheters, such as vascular catheters or the like, in other type or irrigation systems, in tubes containing bacteria sensitive contents, in process industry, in packages, etc.

As discussed in the foregoing, many other materials could be used. For example, it is possible to use other filler materials, with other nanoscale flakes or platelets, than GNP. It is also possible to use other polymer matrix materials than LDPE. It is also possible to use blends of different polymer material as the polymer matrix material. The mixture used for processing may also contain other constituents, e.g. additives such as compatibilizers, stabilizers, antioxidants, plasticizers, etc. The material may also have other antibacterial additives, such as silver or the like. Other ways of removing polymer matrix material at the surface may also be used, in addition to or instead of etching, such as various types of ablation techniques.

It will be appreciated by those versed in the art that several such alternatives similar to those described above could be used without departing from the spirit of the invention, and all such modifications should be regarded as a part of the present invention, as defined in the appended claims.

## Claims

1. A method for producing an antibacterial surface, comprising the steps:
providing a polymer matrix material;
providing a filler material comprising nanoscale flakes or platelets;
processing a mixture of said polymer matrix material and said filler material by pressing said mixture through a die while heated to a temperature above a melting temperature of said polymer matrix material in such a way that the nanoscale flakes or platelets become aligned, with their longitudinal directions being oriented in substantially the same direction;
providing a surface of the processed mixture which is oriented essentially perpendicularly to the longitudinal directions of the nanoscale flakes or platelets;
etching or ablating the surface to partly expose the nanoscale flakes or platelets, thereby making the surface antibacterial.

2. The method of claim 1, wherein the processing of the mixture is at least one of an extrusion process and an injection molding process.

3. The method of claim 1 or 2, wherein the filler material comprises essentially only nanoscale flakes or platelets, and preferably consists of nanoscale flakes or platelets.

4. The method of claim 3, wherein the filler material comprises at least 90 wt-% carbon, and preferably at least 95 wt-%, and more preferably at least 99 wt-%, and most preferably at least 99.9 wt-%.

5. The method of any one of the preceding claims, wherein the mixture comprises an amount of filler material in the range of 3-40 wt-%, and preferably in the range of 5-30 wt-%, and more preferably in the range of 6-25 wt-%, and more preferably in the range of 7.5-20 wt-%, and most preferably in the range of 10-15 wt-%.

6. The method of any one of the preceding claims, wherein the nanoscale flakes or platelets are of graphene or graphite.

7. The method of any one of the preceding claims, wherein the processing provides a homogenous mixture of polymer matrix material and filler material.

8. The method of any one of the preceding claims, wherein the polymer matrix material is a thermoplastic polymer, and preferably a polymer selected from a group consisting of: polycarbonates (PC), polyvinyl chloride (PVC), polyurethanes, copolymers comprising ethylene, such as polyethylene co-acetate, and polyolefin based elastomers, such as polypropylene (PP), styrene-ethylene-butylene-styrene (SEBS) and polyethylene (PE).

9. The method of any one of the preceding claims, wherein the step of providing a surface of the processed mixture comprising cutting of the processed material, and preferably in an essentially transversal or longitudinal direction in relation to a flow direction through the die.

10. The method of any one of the preceding claims, wherein the nanoscale flakes or platelets have an average length in the range of 5-50 microns, and preferably in the range of 10-40 microns, and more preferably in the range of 15-35 microns, and most preferably in the range of 20-30 microns.

11. The method of any one of the preceding claims, wherein the nanoscale flakes or platelets have an average thickness in the range of 0.01-20 nm, and preferably in the range of 0.1-15 nm, and more preferably in the range of 0.5-10 nm, and most preferably in the range of 1-5 nm.

12. The method of any one of the preceding claims, wherein the nanoscale flakes or platelets have an average width in the range of 1-30 microns, and preferably in the range of 1-20 microns, and more preferably in the range of 2-15 microns, and most preferably in the range of 3-15 microns.

13. The method of any one of the preceding claims, wherein the etching or ablating of the surface is made so that the nanoscale flakes or platelets, on an average, extends from the polymer matrix material of the surface by a length in the range of 0.5-30 microns, and preferably in the range of 1-25 microns, and more preferably in the range of 2-20 microns, and most preferably in the range of 3-15 microns.

14. The method of any one of the preceding claims, wherein the distance between any adjacent nanoscale flakes or platelets in the plane of the surface is less than 10 µm, and preferably less than 5 µm, and most preferably less than 1 µm.

15. An article having an antibacterial surface, wherein the article comprises a substrate comprising a polymer matrix and a filler material comprising nanoscale flakes or platelets, wherein said article comprises an antibacterial surface having the nanoscale flakes or platelets arranged essentially aligned to each other and extending out from said surface with a length in the range of 0.5-30 microns.

16. The article of claim 15, wherein the nanoscale flakes or platelets extend out from the surface by a length in the range of 1-25 microns, and more preferably in the range of 2-20 microns, and most preferably in the range of 3-15 microns.

17. The article of any one of the claims 15-16, wherein the article is provided as a coating, and preferably as a coating on a medical device, such as a catheter, and preferably a urinary catheter.
